# EUROPEAN PATENT SPECIFICATION

(11) **EP 3 069 695 B1**
(45) Date of publication and mention of the grant of the patent: **20.12.2017**
(21) Application number: 16275001.2
(22) Date of filing: 04.01.2016
(51) Int. Cl.: A61F 2/966, A61F 2/97

(54) **AN ENDOGRAFT INTRODUCER ASSEMBLY HAVING A TRANSFER SHEATH**
ENDOGRAFTEINFÜHRERANORDNUNG MIT EINER TRANSFERHÜLLE
ENSEMBLE INTRODUCTEUR D'ENDOGREFFE AYANT UNE GAINE DE TRANSFERT

(30) Priority: 18.03.2015 AU 2015201411
(43) Date of publication of application: 21.09.2016
(73) Proprietor: Cook Medical Technologies LLC, Bloomington, IN 47404 (US)
(72) Inventor: DUCKE, Werner, Rochedale South, Queensland 4123 (AU)
(74) Representative: Williams Powell

(56) References cited:
- EP-A1- 2 749 258
- WO-A1-2014/204758

## Description

### TECHNICAL FIELD

The invention relates to medical devices and more particularly to a medical device used for deployment of an intraluminal graft or stent graft, otherwise referred to as an introducer or a stent graft introducer. In particular, this invention relates to an endograft introducer assembly having a transfer sheath.

### BACKGROUND

In the deployment of a graft, or stent graft, into the human or animal body via intraluminal techniques, a deployment device is used to introduce the stent graft into a lumen of the body and, after the stent graft has been deployed and expanded within the lumen, the introducer assembly is retracted.

In some procedures, including some procedures using custom made devices, a main sheath assembly is required to be deployed into the vasculature independently of the graft. In such procedures, after the main sheath assembly is in place, a graft which is pre-compressed into a temporary tube or transfer tube is transferred into a main sheath of the main sheath assembly. At this time it is important that the transfer takes place safely and smoothly. When using currently known endograft introducer assemblies, this results in an additional workload for operating theatre staff and can add risk to the procedure.

EP - A - 2,749,258 discloses an endoluminal prosthesis introducer which includes an elongate tubular sheath including a reinforced longitudinal segment and a peelable longitudinal segment extending distally from the reinforced segment extending distally from the reinforced segment. An elongate tubular cannula may be disposed within the sheath. Upon application of a force applied to a distal end of the sheath, the peelable segment may progressively split in a proximal direction, and the reinforced segment may longitudinally more relative to the cannula in a distal direction.

Throughout this specification, the term distal with respect to a portion of the aorta, a deployment device or an endograft means the end of the aorta, deployment device or endograft further away in the direction of blood flow away from the heart and the term proximal means the portion of the aorta, deployment device or end of the endograft nearer to the heart. When applied to other vessels, similar terms such as caudal and cranial should be understood.

### SUMMARY

Aspects of the present invention are defined in the claims.

According to a first aspect of the invention, there is provided an endograft introducer assembly comprising:
a pusher assembly comprising a guide wire catheter, a tip mounted to a proximal end of the guide wire catheter, a stent graft receiving portion distal of the tip and a pusher distal of the receiving portion;
a transfer sheath extending distally over the stent graft receiving portion from a proximal position adjacent to the tip, the transfer sheath having a first length over the stent graft receiving portion; and
a stopper disposed co-axially around the transfer sheath, the stopper having a proximal end terminating in a stopper surface and a distal portion, the distal portion removably engaged with the transfer sheath so as to prevent relative axial movement between the stopper and the transfer sheath in at least one direction.

Preferred embodiments of the invention address the problem of having to deploy a main sheath assembly into the vasculature independently of a graft, as described hereinbefore, or at least provide the practitioner in the field with a useful alternative device.

In one form, the stopper has a second length, the second length less than the first length.

In one form, the distal portion of the stopper comprises a transfer sheath receiving portion having a receiving outer diameter and wherein the stopper surface has a stopper outer diameter, the stopper outer diameter larger than the receiving outer diameter.

In one form, the stopper has a longitudinal split, the split allowing the stopper to flexibly open for lateral movement over the transfer sheath.

In one form, the transfer sheath comprises a return portion at a distal end thereof, the return portion arranged and constructed to prevent relative axial movement between the stopper and the transfer sheath in the at least one direction.

In one form, the stopper has a first longitudinal stiffness and the transfer sheath has a second longitudinal stiffness, the first longitudinal stiffness greater than the second longitudinal stiffness.

In one form, the transfer sheath is frangible so as to allow it to be removed from the pusher without the need to slide over an end of the pusher.

In one form, the transfer sheath comprises a bifurcated end terminating in a pair of gripable tails.

According to another aspect of the present invention a system comprises: a main sheath assembly having a valve assembly, the valve assembly having a distal end face surrounding an aperture; a pusher assembly comprising a guide wire catheter, a tip mounted to a proximal end of the guide wire catheter, a stent graft receiving portion distal of the tip and a pusher distal of the receiving portion; a transfer sheath extending distally over the stent graft receiving portion from a proximal position adjacent to the tip, the transfer sheath having a first length over the stent graft receiving portion and a stent graft mounted on the receiving portion; and a stopper disposed co-axially around the transfer sheath, the stopper having a proximal end terminating in a stopper surface and a distal portion, the distal portion removably engaged with the transfer sheath so as to prevent relative axial movement between the stopper and the transfer sheath in at least one direction, wherein, in use, when an operator pushes the pusher axially towards the main sheath assembly so that the stopper surface abuts the distal end face of the valve assembly the stopper does not pass into the valve assembly and a reaction force is transmitted from the stopper surface along the stopper to the proximal end of the transfer sheath, such that the transfer sheath is held at a pre-determined position inside the valve assembly.

The stopper may have a first longitudinal stiffness and the transfer sheath may have a second longitudinal stiffness, the first longitudinal stiffness may be greater than the second longitudinal stiffness. The distal portion of the stopper may comprise a transfer sheath receiving portion having a receiving outer diameter. The stopper surface may have a stopper outer diameter.The stopper outer diameter may be larger than the receiving outer diameter. The transfer sheath imay be frangible so as to allow it to be removed from the pusher without the need to slide over an end of the pusher. The transfer sheath may comprise a bifurcated end terminating in a pair of gripable tails.

### BRIEF DESCRIPTION OF DRAWINGS

Embodiments of the present invention are described below, by way of example only, with reference to the accompanying drawings, wherein:
Figure 1A is an isometric view of an endograft introducer assembly according to the invention;
Figure 1B is an isometric view of a pusher assembly which forms part of the endograft introducer assembly of Figure 1A.
Figure 2 is a close up view of a proximal end of the introducer assembly of Figure 1A;Figure 3 is a similar view to that of Figure 2, but shows a guide wire catheter and tip, also shown in Figures 1 and 2, in a straightened condition;
Figure 4 is a similar view to that of Figure 3, but shows the introducer assembly of Figures 1 to 3 loaded with a stent graft;
Figures 5 and 6 are diagrammatic views showing a portion of the distal end of the introducer assembly shown in Figure 4, but with the stent graft retracted and covered by a transfer sheath;
Figure 7A is a detailed isometric view of a stopper, the stopper being a component of the introducer assembly shown in Figures 1 to 6;
Figures 7B and 7C show alternative embodiments of the stopper shown in Figure 7A;
Figure 8 shows an isometric view of the introducer assembly of Figures 1 to 6 on a guide wire being guided towards a main sheath assembly;
Figure 9 is a close up view of a portion of the main sheath assembly shown in Figure 8; and
Figure 10 is a similar view to that of Figure 8 but is a close up view and shows the stopper of the introducer assembly abutting the main sheath assembly;
Figure 11A shows a diagrammatic end view of a stopper of the introducer assembly of Figures 1 to 3 in an installed condition and being removed respectively.
Figure 11B is a similar view to that of Figure 11A, but shows the stopper being removed.

The components in the drawings are not necessarily to scale, emphasis instead being placed upon illustrating the principles of the teachings herein.

### DESCRIPTION OF EMBODIMENTS

Aspects of the present invention are defined in the appended claims.

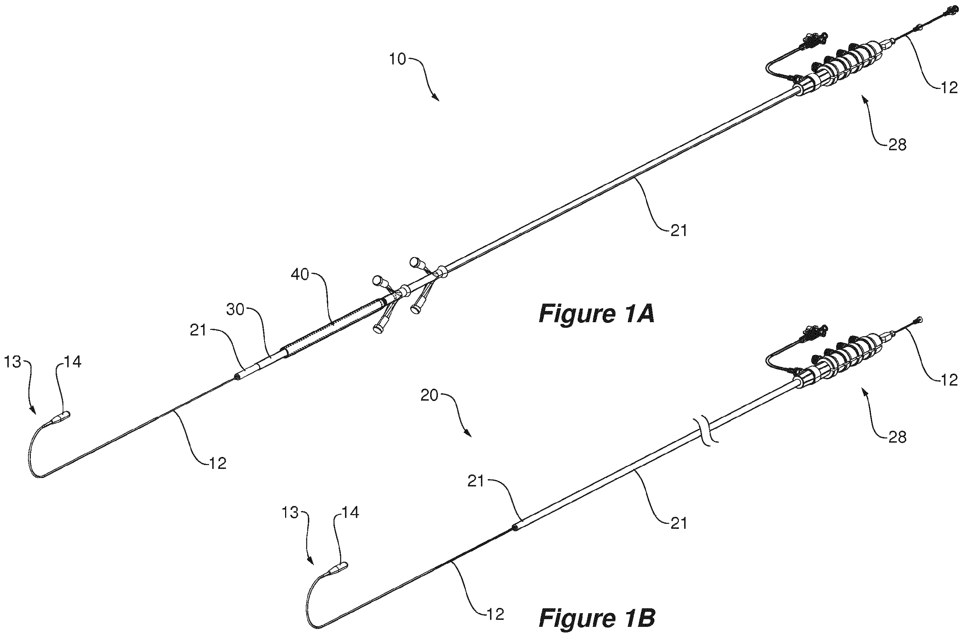

Referring to Figure 1A, an endograft introducer assembly 10 according to the invention is shown. The endograft introducer assembly 10 includes a pusher assembly 20, as is shown in Figure 1A. The pusher assembly 20 comprises a guide wire catheter 12, a tip 14 mounted to a proximal end 13 of the guide wire catheter 12 and a pusher 21. The pusher 21 has a handle 28.

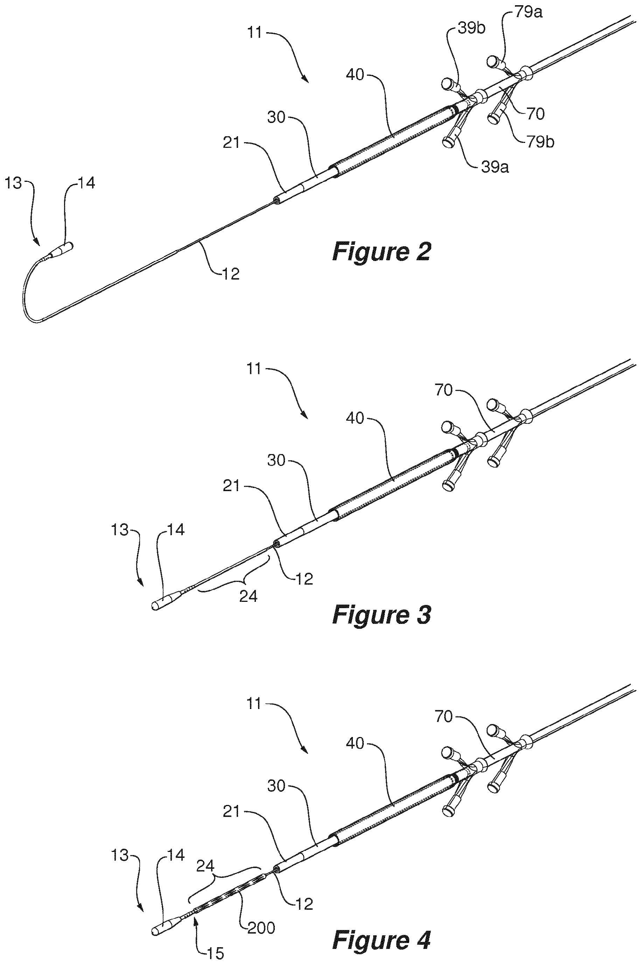

Referring now to Figures 2 and 3, a proximal end 11 of the endograft introducer assembly 10 is shown more clearly. Figure 3 shows a stent graft receiving portion 24 and shows that the pusher 21 is distal of the stent graft receiving portion 24.

Referring now to Figure 4, a stent graft 200 is shown mounted on the receiving portion 24. Figure 4 also shows a transfer sheath 30.

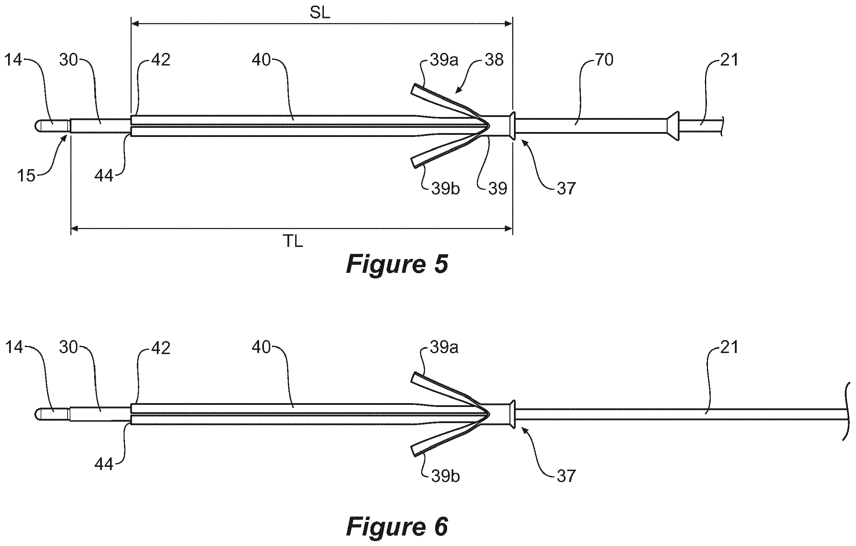

Turning now to Figure 5, the transfer sheath 30 is in a position extending distally over the stent graft 200 from a proximal position 15 adjacent to the tip 14.

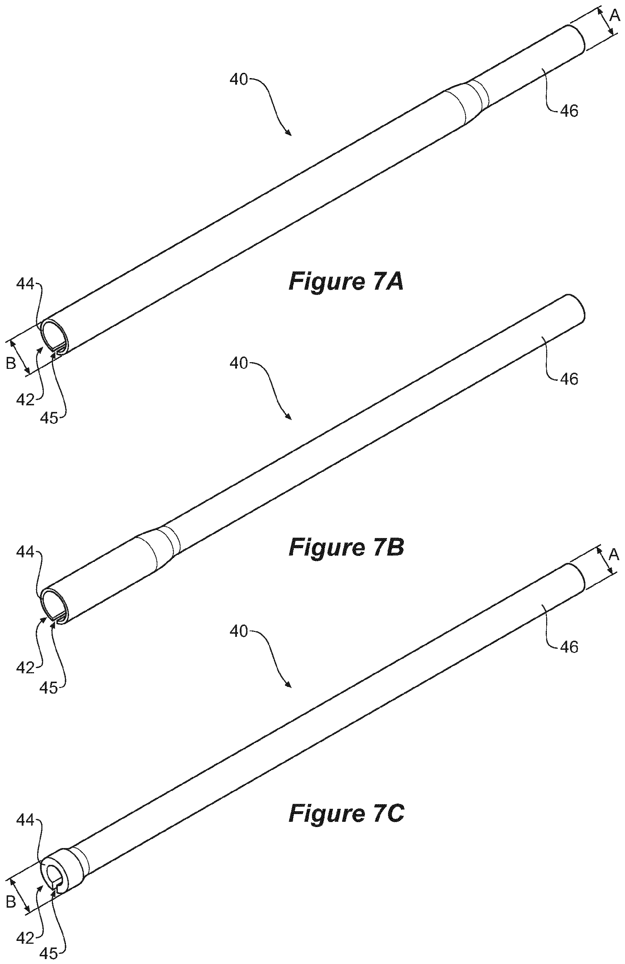

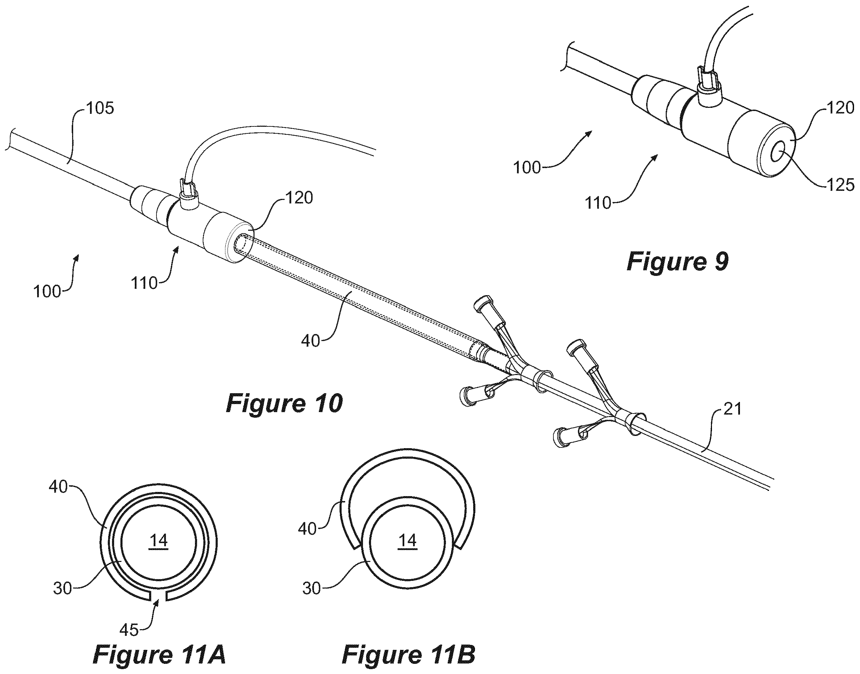

Again referring to Figure 5, a stopper 40 disposed coaxially around the transfer sheath 30 is shown. The stopper 40 has a proximal end 42 terminating in a stopper surface 44. This stopper surface 44 is more clearly shown in Figure 7A. Figure 7A also shows that the stopper 40 has a distal portion 46, the distal portion 46 being removably engaged with the transfer sheath 30, as is shown in Figures 4 and 5 for instance, so as to prevent relative movement between the stopper 40 and the transfer sheath 30 in at least one direction. More specifically, when the stopper surface 44 of the stopper 40 is pushed against a surface, such as the distal end face 120 of the valve assembly 110 shown in Figures 9 and 10, the reaction force is transferred along the a stopper 40 to its distal portion 46 thereby preventing movement of the transfer sheath 30 towards the distal end face 120 (which functions as a reaction surface) as the pusher 21 is pushed in a direction towards the distal end face 120.

The stopper 40 shown in Figure 7A and Figure 11A has a longitudinal split 45, the split 45 allowing the stopper 40 to flexibly open for lateral movement over the transfer sheath 30. Figure 11B shows the stopper forced open for removal from around the transfer sheath 30.

In other embodiments of the invention, alternative stoppers can be used. For instance, the stopper 40 illustrated in Figure 7B or the stopper 40 illustrated in Figure 7C can be used.

The transfer sheath 30 has a first length TL, as is illustrated in Figure 5. This length is sufficient to entirely cover the stent graft 200 illustrated in Figure 4. The stopper 40, also illustrated in Figure 5, has a second length SL. This second length, SL, is less than the first length. The reason for this becomes apparent when use of the introducer assembly 10 is explained (see below).

Referring again to Figure 7A, it can be seen that the distal portion 46 of the stopper 40 has a receiving outer diameter A. This receiving outer diameter A receives a return portion 39 of the transfer sheath 30, as is shown in Figure 5. The return portion 39 is arranged and constructed to prevent relative axial movement between the stopper 40 and the transfer sheath 30 in one direction.

The receiving outer diameter A is smaller than the stopper surface 44 outer diameter B, as indicated on Figure 7A. This allows the stopper 40 to snuggly fit around the transfer sheath 30 at its distal end and to loosely fit around the transfer sheath 30 at, or towards, its distal end 37, where the stopper 40 has a constant wall thickness.

The transfer sheath 30 is frangible so as to allow it to be removed from the pusher 21 without the need to be slid over an end of the pusher 21. The transfer sheath 30 comprises a bifurcated end 38 terminating in a pair of gripable tails 39a and 39b as can be seen in Figure 2. These gripable tails 39a and 39b allow the operator to peel and remove the transfer sheath 30 from the pusher assembly 20. The transfer sheath 30 can thus be considered a "peelaway" component.

Figures 1 and 5 show a peelaway plug 70. This peelaway plug 70 is provided to plug the distal end 37 of the transfer sheath 30. This ensures that the introducer assembly 10 can be flushed with a flushing fluid. The peelaway plug 70 may include gripable tails 79a and 79b, as illustrated in Figure 2. Alternative plugs, such as the plug 70 schematically illustrated in Figure 5, may be used.

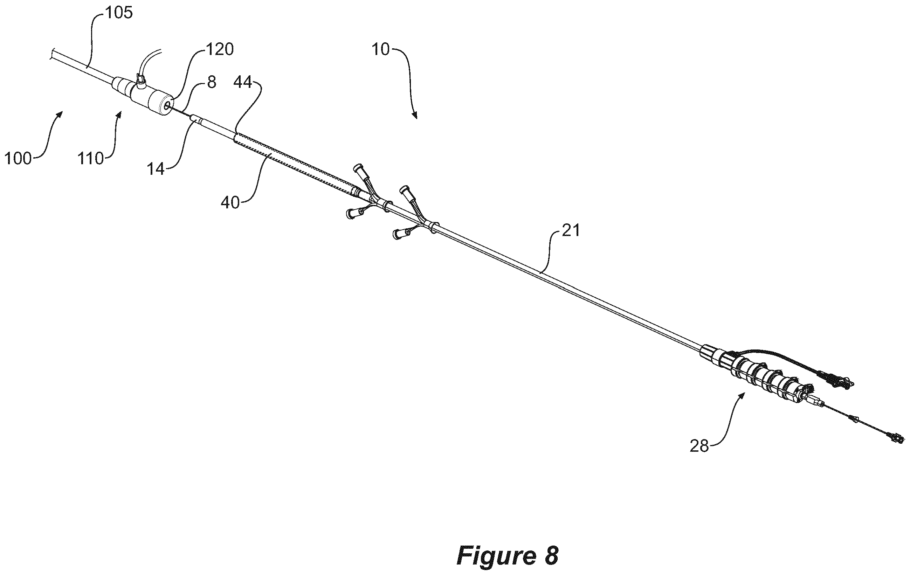

Referring to Figure 8, the introducer assembly 10 is shown over a guide wire 8 adjacent a main sheath assembly 100. Figure 9 shows that the main sheath assembly 100 has a valve assembly 110 that has a distal end face 120 surrounding an aperture 125. Within the aperture 125 is a valve as is known in the art. The valve prevents, or at least minimises, blood leakage. Figure 10 shows the stopper 40 with its stopper surface 44 (shown in Figure 8) abutting the distal end face 120 of the valve assembly 110. As mentioned above, the diameter B is larger than the diameter A, so as to ensure that the stopper surface 44 does not enter the aperture 125, and instead firmly abuts the annular distal end face 120, as is shown in Figure 10.

The stopper 40 has a first longitudinal stiffness and the transfer sheath 30 has a second longitudinal stiffness. The first longitudinal stiffness is greater than the second longitudinal stiffness. The longitudinal stiffness of the stopper 40 is such that when a person pushes the pusher 21 axially along the guide wire 8, as shown in Figure 8, towards the main sheath assembly 100 so that the stopper surface 44 abuts the distal end face 120, a reaction force is transmitted from the stopper surface 44 along the stopper to the proximal end 38 of the transfer sheath 30. This reaction force is sufficient to prevent further movement of the transfer sheath 30 and thereby only allows the transfer sheath 30 to travel a small distance into the main sheath assembly 100. This distance is the difference between the lengths TL and SL, as illustrated in Figure 5. As the pusher 21 is further pushed into the main sheath assembly 100, the stent graft 200 begins to move axially with respect to the transfer sheath 30 such that it is transferred from the transfer sheath 30 into the main sheath 105. This prevents the transfer sheath 30 from progressing too far into the valve assembly 110 and hence prevents the transfer sheath 30 becoming jammed in the valve within the valve assembly 110. The relative length of the stopper 40 and the temporary transfer sheath 30 is such that the transfer sheath 30 is held at a pre-determined position inside the valve assembly 110, so as to prevent both the possibility of graft deployment within the valve assembly 110 and the possibility of the transfer sheath 30 becoming jammed inside the valve assembly 110. The longitudinal slit 45 is provided so that the stopper 40 is easily removable when no longer required. Figure 11A shows the stopper 40 of Figure 7A around the transfer sheath 30 and Figure 11B shows the stopper 40 of Figure 7A being removed from the transfer sheath 30.The material thickness and properties of the stopper 40 are such that, even with the longitudinal split 45, it is positively located so that it is not easily dislodged accidently from its coaxial position around the transfer sheath 30.

An advantage of the arrangement described above is that its operators do not require as much training and do not need as much skill to transfer a graft 200 into a main sheath 100 during and endovascular procedure. Furthermore, the likelihood of jamming is reduces, as described above.

Throughout the specification and the claims that follow, unless the context requires otherwise, the words "comprise" and "include" and variations such as "comprising" and "including" will be understood to imply the inclusion of a stated integer or group of integers, but not the exclusion of any other integer or group of integers.

The reference to any prior art in this specification is not, and should not be taken as, an acknowledgement of any form of suggestion that such prior art forms part of the common general knowledge.

It will be appreciated by those skilled in the art that the invention is not restricted in its use to the particular application described. Neither is the present invention restricted in its preferred embodiment with regard to the particular elements and/or features described or depicted herein. It will be appreciated that the invention is not limited to the embodiment or embodiments disclosed, but is capable of numerous rearrangements, modifications and substitutions without departing from the scope of the invention as set forth and defined by the following claims.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

## Claims

1. An endograft introducer assembly (10) comprising:
a pusher assembly (20) comprising a guide wire catheter (12), a tip (14) mounted to a proximal end (13) of the guide wire catheter (12), a stent graft receiving portion (24) distal of the tip and a pusher (21) distal of the receiving portion;
a transfer sheath (30) extending distally over the stent graft receiving portion (24) from a proximal position adjacent to the tip (14), the transfer sheath (30) having a first length over the stent graft receiving portion; and
a stopper (40) disposed co-axially around the transfer sheath (30), the stopper (40) having a proximal end (42) terminating in a stopper surface (44) and a distal portion (46), the distal portion (46) removably engaged with the transfer sheath (30) so as to prevent relative axial movement between the stopper (40) and the transfer sheath (30) in at least one direction,
wherein the distal portion (46) of the stopper (40) comprises a transfer sheath receiving portion having a receiving outer diameter (B) and wherein the stopper surface (44) has a stopper outer diameter (B), the stopper outer diameter larger than the receiving outer diameter (A).

2. An introducer assembly according to claim 1 comprising a stent graft (200) mounted on the receiving portion.

3. The introducer assembly of any preceding claim wherein the stopper (40) has a second length, the second length less than the first length.

4. The introducer assembly of any preceding claim wherein the stopper (40) has a longitudinal split (45), the split allowing the stopper (40) to flexibly open for lateral movement over the transfer sheath.

5. The introducer assembly of any preceding claim wherein the transfer sheath (30) comprises a return portion at a distal end thereof, the return portion arranged and constructed to prevent relative axial movement between the stopper (40) and the transfer sheath (30) in the at least one direction.

6. The introducer assembly of any preceding claim wherein the stopper has a first longitudinal stiffness and the transfer sheath (30) has a second longitudinal stiffness, the first longitudinal stiffness greater than the second longitudinal stiffness.

7. The introducer assembly of any preceding claim wherein the transfer sheath (30) is frangible so as to allow it to be removed from the pusher without the need to slide over an end of the pusher.

8. The introducer assembly of any preceding claim wherein the transfer sheath (30) comprises a bifurcated end terminating in a pair of gripable tails (39a, 39b).

9. A system comprising:
a main sheath assembly (100) having a valve assembly (110), the valve assembly (110) having a distal end face (120) surrounding an aperture (125);
a pusher assembly (20) comprising a guide wire catheter (12), a tip (14) mounted to a proximal end (13) of the guide wire catheter (12), a stent graft receiving portion distal of the tip and a pusher (21) distal of the receiving portion;
a transfer sheath (30) extending distally over the stent graft receiving portion (24) from a proximal position adjacent to the (14) tip, the transfer sheath (30) having a first length over the stent graft receiving portion and a stent graft mounted on the receiving portion; and
a stopper (40) disposed co-axially around the transfer sheath (30), the stopper (40) having a proximal end terminating in a stopper surface (44) and a distal portion (46), the distal portion removably engaged with the transfer sheath (30) so as to prevent relative axial movement between the stopper (40) and the transfer sheath (30) in at least one direction,
wherein, in use, when an operator pushes the pusher (21) axially towards the main sheath assembly (100) so that the stopper surface (44) abuts the distal end face (120) of the valve assembly the stopper (40) does not pass into the valve assembly and a reaction force is transmitted from the stopper surface (44) along the stopper (44) to the proximal end of the transfer sheath (30), such that the transfer sheath (30) is held at a pre-determined position inside the valve assembly.

10. The assembly of claim 9 wherein the stopper (40) has a first longitudinal stiffness and the transfer sheath (30) has a second longitudinal stiffness, the first longitudinal stiffness greater than the second longitudinal stiffness.

11. The assembly of any of claims 9 and 10 wherein the distal portion of the stopper (40) comprises a transfer sheath receiving portion having a receiving outer diameter and wherein the stopper surface has a stopper outer diameter, the stopper outer diameter larger than the receiving outer diameter.

12. The assembly of any of claims 9 to 11 wherein the transfer sheath (30) is frangible so as to allow it to be removed from the pusher without the need to slide over an end of the pusher.

13. The assembly of any of claims 9 to 12 wherein the transfer sheath (30) comprises a bifurcated end terminating in a pair of gripable tails.

## Patentansprüche

1. Endotransplantateinführbaugruppe (10), umfassend:
eine Drückerbaugruppe (20), umfassend einen Führungsdrahtkatheter (12), eine Spitze (14), die an dem proximalen Ende (13) des Führungsdrahtkatheters (12) angebracht ist, einen Stenttransplantataufnahmeteil (24) distal bezogen auf die Spitze und einen Drücker (21) distal bezogen auf den Aufnahmeteil;
eine Überführungshülle (30), die von einer proximalen Position nahe der Spitze (14) distal über den Stenttransplantataufnahmeteil (24) verläuft, wobei die Überführungshülle (30) eine erste Länge über den Stenttransplantataufnahmeteil aufweist; und
einen Stopper (40), der koaxial um die Überführungshülle (30) angeordnet ist, wobei der Stopper (40) ein proximales Ende (42), das an einer Stopperfläche (44) endet, und einen distalen Teil (46) aufweist, wobei der distale Teil (46) entfernbar mit der Überführungshülle (30) in Eingriff steht, um relative Axialbewegung zwischen dem Stopper (40) und der Überführungshülle (30) in wenigstens eine Richtung zu verhindern,
wobei der distale Teil (46) des Stoppers (40) einen Überführungshüllenaufnahmeteil umfasst, der einen aufnehmenden Außendurchmesser (B) aufweist, und wobei die Stopperfläche (44) einen Stopperaußendurchmesser (B) aufweist, wobei der Stopperaußendurchmesser größer als der aufnehmende Außendurchmesser (A) ist.

2. Einführbaugruppe gemäß Anspruch 1, umfassend ein Stenttransplantat (200), das an dem Aufnahmeteil angebracht ist.

3. Einführbaugruppe gemäß einem der vorstehenden Ansprüche, wobei der Stopper (40) eine zweite Länge aufweist, wobei die zweite Länge kleiner als die erste Länge ist.

4. Einführbaugruppe gemäß einem der vorstehenden Ansprüche, wobei der Stopper (40) einen Längsschlitz (45) aufweist, wobei der Schlitz erlaubt, dass sich der Stopper (40) zur seitlichen Bewegung über die Überführungshülle flexibel öffnet.

5. Einführbaugruppe gemäß einem der vorstehenden Ansprüche, wobei die Überführungshülle (30) einen Rückführteil an ihrem distalen Ende umfasst, wobei der Rückführteil angeordnet und gestaltet ist, relative Axialbewegung zwischen dem Stopper (40) und der Überführungshülle (30) in der wenigstens einen Richtung zu verhindern.

6. Einführbaugruppe gemäß einem der vorstehenden Ansprüche, wobei der Stopper eine erste Längssteifigkeit aufweist und die Überführungshülle (30) eine zweite Längssteifigkeit aufweist, wobei die erste Längssteifigkeit größer als die zweite Längssteifigkeit ist.

7. Einführbaugruppe gemäß einem der vorstehenden Ansprüche, wobei die Überführungshülle (30) brechbar ist, so dass sie von dem Drücker entfernt werden kann, ohne über ein Ende des Drückers gleiten zu müssen.

8. Einführbaugruppe gemäß einem der vorstehenden Ansprüche, wobei die Überführungshülle (30) ein gegabeltes Ende umfasst, das in einem Paar von greifbaren Endstücken (39a, 39b) endet.

9. System, umfassend:
eine Haupthüllenbaugruppe (100) mit einer Ventilbaugruppe (110), wobei die Ventilbaugruppe (110) eine distale Endfläche (120) aufweist, die eine Öffnung (125) umgibt;
eine Drückerbaugruppe (20), umfassend einen Führungsdrahtkatheter (12), eine Spitze (14), die an dem proximalen Ende (13) des Führungsdrahtkatheters (12) angebracht ist, einen Stenttransplantataufnahmeteil distal bezogen auf die Spitze und einen Drücker (21) distal bezogen auf den Aufnahmeteil;
eine Überführungshülle (30), die von einer proximalen Position nahe der Spitze (14) distal über den Stenttransplantataufnahmeteil (24) verläuft, wobei die Überführungshülle (30) eine erste Länge über den Stenttransplantataufnahmeteil aufweist und ein Stenttransplantat an dem Aufnahmeteil angebracht ist; und
einen Stopper (40), der koaxial um die Überführungshülle (30) angeordnet ist, wobei der Stopper (40) ein proximales Ende, das an einer Stopperfläche (44) endet, und einen distalen Teil (46) aufweist, wobei der distale Teil entfernbar mit der Überführungshülle (30) in Eingriff steht, um relative Axialbewegung zwischen dem Stopper (40) und der Überführungshülle (30) in wenigstens eine Richtung zu verhindern,
wobei in Verwendung ein Bediener den Drücker (21) axial zu der Haupthüllenbaugruppe (100) drückt, so dass die Stopperfläche (44) an die distale Endfläche (120) der Ventilbaugruppe anstößt, der Stopper (40) nicht in die Ventilbaugruppe eintritt und eine Reaktionskraft von der Stopperfläche (44) entlang des Stoppers (44) zu dem proximalen Ende der Überführungshülle (30) übertragen wird, so dass die Überführungshülle (30) an einer vorbestimmten Stelle im Inneren der Ventilbaugruppe gehalten wird.

10. Baugruppe gemäß Anspruch 9, wobei der Stopper (40) eine erste Längssteifigkeit aufweist und die Überführungshülle (30) eine zweite Längssteifigkeit aufweist, wobei die erste Längssteifigkeit größer als die zweite Längssteifigkeit ist.

11. Baugruppe gemäß einem der Ansprüche 9 und 10, wobei der distale Teil des Stoppers (40) einen Überführungshüllenaufnahmeteil umfasst, der einen aufnehmenden Außendurchmesser aufweist, und wobei die Stopperfläche einen Stopperaußendurchmesser aufweist, wobei der Stopperaußendurchmesser größer als der aufnehmende Außendurchmesser ist.

12. Baugruppe gemäß einem der Ansprüche 9 bis 11, wobei die Überführungshülle (30) brechbar ist, so dass sie von dem Drücker entfernt werden kann, ohne über ein Ende des Drückers gleiten zu müssen.

13. Baugruppe gemäß einem der Ansprüche 9 bis 12, wobei die Überführungshülle (30) ein gegabeltes Ende umfasst, das in einem Paar von greifbaren Endstücken (39a, 39b) endet.

## Revendications

1. Ensemble introducteur (10) d'endogreffe, comprenant :
un ensemble poussoir (20), comprenant un cathéter (12) à fil guide, un embout (14) monté sur l'extrémité proximale (13) du cathéter (12) à fil guide, une partie réceptrice (24) de greffon de stent distale de l'embout et un poussoir (21) distal de la partie réceptrice ;
une gaine de transfert (30) s'étendant distalement sur la partie réceptrice (24) de greffon de stent à partir d'une position proximale adjacente à l'embout (14), la gaine de transfert (30) comportant une première longueur sur la partie réceptrice de greffon de stent ; et
un bouchon (40) disposé coaxialement autour de la gaine de transfert (30), le bouchon (40) comportant une extrémité proximale (42) se terminant dans une surface (44) de bouchon et une partie distale (46), la partie distale (46) étant en prise amovible avec la gaine de transfert (30) de façon à empêcher un mouvement axial relatif entre le bouchon (40) et la gaine de transfert (30) dans au moins une direction, dans lequel la partie distale (46) du bouchon (40) comprend une partie réceptrice de gaine de transfert ayant un diamètre extérieur (B) de réception et dans lequel la surface (44) de bouchon a un diamètre extérieur (B) de bouchon, le diamètre extérieur de bouchon étant supérieur au diamètre extérieur (A) de réception.

2. Ensemble introducteur selon la revendication 1, comprenant un greffon de stent (200) monté sur la partie réceptrice.

3. Ensemble introducteur selon l'une quelconque des revendications précédentes, dans lequel le bouchon (40) a une seconde longueur, la seconde longueur étant inférieure à la première longueur.

4. Ensemble introducteur selon l'une quelconque des revendications précédentes, dans lequel le bouchon (40) comporte une fente (45) longitudinale, la fente permettant au bouchon (40) de s'ouvrir souplement pour effectuer un mouvement latéral sur la gaine de transfert.

5. Ensemble introducteur selon l'une quelconque des revendications précédentes, dans lequel la gaine de transfert (30) comprend une partie de retour de son extrémité distale, la partie de retour étant agencée et structurée pour empêcher un mouvement axial relatif entre le bouchon (40) et la gaine de transfert (30) dans ladite direction.

6. Ensemble introducteur selon l'une quelconque des revendications précédentes, dans lequel le bouchon présente une première raideur longitudinale et la gaine de transfert (30) présente une seconde raideur longitudinale, la première raideur longitudinale étant supérieure à la seconde raideur longitudinale.

7. Ensemble introducteur selon l'une quelconque des revendications précédentes, dans lequel la gaine de transfert (30) est cassante afin de permettre de la retirer du poussoir sans qu'il soit qu'il soit nécessaire de coulisser sur une extrémité du poussoir.

8. Ensemble introducteur selon l'une quelconque des revendications précédentes, dans lequel la gaine de transfert (30) comprend une extrémité à deux branches se terminant par une paire de queues (39a, 39b) pouvant être saisies.

9. Système comprenant :
un ensemble gaine principale (100) comportant un ensemble valve (110), l'ensemble valve (110) comportant une face terminale distale (120) entourant une ouverture (125) ;
un ensemble poussoir (20) comprenant un cathéter (12) à fil guide, un embout (14) monté sur l'extrémité proximale (13) du cathéter (12) à fil guide, une partie réceptrice de greffon de stent distale de l'embout et un poussoir (21) distal de la partie réceptrice ;
une gaine de transfert (30) s'étendant distalement sur la partie réceptrice (24) de greffon de stent à partir d'une position proximale adjacente à l'embout (14), la gaine de transfert (30) comportant une première longueur sur la partie réceptrice de greffon de stent et un greffon de stent monté sur la partie réceptrice ; et
un bouchon (40) disposé coaxialement autour de la gaine de transfert (30), le bouchon (40) comportant une extrémité proximale se terminant dans une surface (44) de bouchon et une partie distale (46), la partie distale étant en prise amovible avec la gaine de transfert (30) de façon à empêcher un mouvement axial relatif entre le bouchon (40) et la gaine de transfert (30) dans au moins une direction,
dans lequel, lors de l'utilisation, quand un opérateur appuie axialement sur le poussoir (21) vers l'ensemble gaine principale (100) de façon à ce que la surface (44) de bouchon vienne en butée contre la face terminale distale (120) de l'ensemble valve, le bouchon (40) ne pénètre pas dans l'ensemble valve et une force de réaction est transmise de la surface (44) de bouchon le long du bouchon (44) à l'extrémité proximale de la gaine de transfert (30), de telle sorte que la gaine de transfert (30) est maintenue à une position prédéterminée à l'intérieur de l'ensemble valve.

10. Ensemble selon la revendication 9, dans lequel le bouchon (40) présente une première raideur longitudinale et la gaine de transfert (30) présente une seconde raideur longitudinale, la première raideur longitudinale étant supérieure à la seconde raideur longitudinale.

11. Ensemble selon la revendication 9 ou 10, dans lequel la partie distale du bouchon (40) comprend une partie réceptrice de gaine de transfert ayant un diamètre extérieur de réception et dans lequel la surface de bouchon a un diamètre extérieur de bouchon, le diamètre extérieur de bouchon étant supérieur au diamètre extérieur de réception.

12. Ensemble selon l'une quelconque des revendications 9 à 11, dans lequel la gaine de transfert (30) est cassante afin de permettre de la retirer du poussoir sans qu'il soit qu'il soit nécessaire de coulisser sur une extrémité du poussoir.

13. Ensemble selon l'une quelconque des revendications 9 à 12, dans lequel la gaine de transfert (30) comprend une extrémité à deux branches se terminant par une paire de queues pouvant être saisies.
